# EUROPEAN PATENT SPECIFICATION

(11) **EP 2 263 751 B1**
(45) Date of publication and mention of the grant of the patent: **09.07.2014**
(21) Application number: 10174276.5
(22) Date of filing: 10.10.2008
(51) Int. Cl.: A61P 35/00, A61K 31/5377

(54) **Isoxazole compound for the treatment of gastrointestinal cancers**
Isoxazolverbindung für die Behandlung von Magen-Darm Krebsarten
Composé isoxazole pour le traitement de cancers gastrointestinaux

(30) Priority: 12.10.2007 EP 07118421
(43) Date of publication of application: 22.12.2010
(62) Divisional of application: 08836801.4
(73) Proprietor: Novartis AG, 4056 Basel (CH)
(72) Inventor: Chene, Patrick, 68100, Mulhouse (FR); Garcia-Echeverria, Carlos, 92210, Saint-Cloud (FR); Jensen, Michael Rugaard, 4054, Basel (CH); Quadt, Cornelia, 4123, Allschwil (CH); Radimerski, Thomas, 4492, Tecknau (CH); Schoepfer, Joseph, 4002, Basel (CH)
(74) Representative: Roth, Peter Richard

(56) References cited:
- WO-A-2004/072051
- WO-A-2006/113498
- WO-A1-2008/104595
- US-A1- 2007 105 862
- DATABASE ADISCTI [Online] ADIS; 13 September 2007 (2007-09-13), US NATIONAL INSTITUTES OF HEALTH: "AUY922 : therapeutic use Advanced breast cancer Phase I/II trial in patients with either HER2-positive or oestrogen receptor-positive locally advanced or metastatic disease" XP002466385 retrieved from STN Database accession no. 2007:8882

## Description

The invention relates to the use of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for the manufacture of pharmaceutical compositions for use in the treatment of cancer of the colon, the liver, the pancreas, the stomach, gastrointestinal tract, e.g. gastrointestinal stromal tumor, to 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for use in the treatment of cancer of the colon, the liver, the pancreas, the stomach, gastrointestinal tract, e.g. gastrointestinal stromal tumor. Further described is the treatment of warm-blooded animals including humans suffering from cancer of the colon, the liver, the pancreas, the stomach, gastrointestinal tract, e.g. gastrointestinal stromal tumor, by administering to said animal in need of such treatment an effective dose of 5-(2,4-Dihydroxy-5-isopropyl-phenyl)-4-(4-morpholin-4-ylmethyl-phenyl)-isoxazole-3-carboxylic acid ethylamide or a pharmaceutically acceptable salt or a hydrate or a solvate.

Management of cancers of the bladder, the colon, the liver, the lung, e.g. pleuramesothelioma, e.g. non small cell, e.g. small cell, the breast, the ovaries, the pancreas, the kidney, the stomach, gastrointestinal tract, e.g. gastrointestinal stromal tumor, the prostate, the head and neck, the brain, e.g. glioblastoma, blood, e.g. hematological cancer, e.g. leukemia, e.g. acute myeloid leukemia, e.g. multiple myeloma and/or melanomas a major problem.

Heat shock protein 90 (Hsp90) is recognized as a new anti-cancer target. Hsp90 is a ubiquitous, highly abundant (1-2% of the total cellular protein), essential protein which functions as a molecular chaperone to ensure the conformational stability, shape and function of client proteins. Inhibition of its intrinsic ATPase activity of Hsp90 disrupts the Hsp90-client protein interaction resulting in their degradation via the ubiquitin proteasome pathway. A subset of Hsp90 client proteins, such as Raf, AKT, CDK4 and the EGFR family including ErbB2 are oncogenic signaling molecules critically involved in cell growth, differentiation and apoptosis, processes which are fundamentaly important in cancer cells. The simultaneous degradation of multiple oncoproteins is believed to produce the antitumor effects observed with Hsp90 inhibitors.

The Hsp90 family of chaperones is comprised of four members: Hsp90α and Hsp90β both located in the cytosol, GRP94 in the endoplasmic reticulum, and TRAP1 in the mitochondria (Csermely et al., 1998). Hsp90 (unless otherwise indicated, Hsp90 refers to Hsp90α and Hsp90β) is the most abundant cellular chaperone, constituting about 1% - 2% of total protein (Jakob and Buchner, 1994). Among the stress proteins, Hsp90 is unique because it is not required for the biogenesis of most polypeptides (Nathan et al., 1997). Its cellular targets, also called client proteins, are conformationally labile signal transducers that play a critical role in growth control, cell survival and tissue development (Pratt and Toft, 2003).

Hsp90 chaperones, which possess a conserved ATP-binding site at their N-terminal domain (Chene, 2002) belong to a small ATPase sub-family known as the DNA Gyrase, Hsp90, Histidine Kinase and MutL (GHKL) sub-family (Dutta and Inouye, 2000). The chaperoning (folding) activity of Hsp90 depends on its ATPase activity which is weak for the isolated enzyme. However, it has been shown that the ATPase activity of Hsp90 is enhanced upon its association with proteins known as co-chaperones (Kamal et al., 2003). Therefore, *in vivo*, Hsp90 proteins work as subunits of large, dynamic protein complexes. Hsp90 is essential for eukaryotic cell survival and is overexpressed in many tumors.

5-(2,4-Dihydroxy-5-isopropyl-phenyl)-4-(4-morpholin-4-ylmethyl-phenyl)-isoxazole-3-carboxylic acid ethylamide is an Hsp90 inhibitor, its synthesis is described for instance in WO 2004/072051, example 78.

Surprisingly it has now been found that 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate is useful in the treatment of cancer of the colon, the liver, the pancreas, the stomach, gastrointestinal tract, e.g. gastrointestinal stromal tumor.

Accordingly the present invention provides the use of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for the manufacture of pharmaceutical compositions for use in the treatment of cancer of the colon, the liver, the pancreas, the stomach, the gastrointestinal tract, e.g. gastrointestinal stromal tumor.

There is further described the treatment of humans suffering from cancer of the colon, the liver, the pancreas, the stomach, gastrointestinal tract, e.g. gastrointestinal stromal tumor, which comprises administering to said human in need of such treatment a dose of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate effective against cancer of the colon, the liver, the pancreas, the stomach, gastrointestinal tract, e.g. gastrointestinal stromal tumor.

In a further aspect the present invention provides a pharmaceutical preparation for use in the treatment of cancer of the colon, the liver, the pancreas, the stomach, gastrointestinal tract, e.g. gastrointestinal stromal tumor, comprising 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate and at least one pharmaceutically acceptable carrier.

Depending on species, age, individual condition, mode of administration, and the clinical picture in question, effective doses for example weekly doses of about 2 to 300 mg, preferably 50 to 160 mg of 5-(2,4-Dihydroxy-5-isopropyl-phenyl)-4-(4-morpholin-4-ylmethyl-phenyl)-isoxazole-3-carboxylic acid ethylamide are administered to a human.

There is further disclosed a method for administering to a human having having cancer of the colon, the liver, the pancreas, the stomach, the gastrointestinal tract, e.g. gastrointestinal stromal tumor, 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate, which comprises administering a pharmaceutically effective amount of 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate to a human subject about once weekly.

Following is a description by way of example only.

### Example 1: In vitro effects of NVP-AUY922 on a panel of tumor derived cell lines.

Thirty-seven cancer derived cell lines are used (BT474, MDA-MB-361, MDA-MB-453, SKBr3, T47D, MCF7, MDA-MB-231, MDA-MB-468, SK-MEL-5, A375, MALME-3M, SK-MEL-28, WM266.4, RPM18226, U266, BE, Colo205, HCT116, HT29, MAWI, RKO, U87MG, HN5, RPMI-8226, A549, MV522, NCI-H1299, NCI-H460, 41 M, A2780, CH1, NCI-N87, SKOV3, PC3, MO7e, GIST882 and Baf3) to test the effect of 5-(2,4-Dihydroxy-5-isopropyl-phenyl)-4-(4-morpholin-4-ylmethyl-phenyl)-isoxazole-3-carboxylic acid ethylamide. Cell lines are commercially available from American Type Culture Collection (ATCC). These cell lines cover the following 12 cancer or tumor types: breast, melanoma, multiple myeloma (MM), colon, glioblastoma, head & neck, leukemia, lung, ovarian, prostate, stomach and gastrointestinal stromal tumour (GIST). After division and medium change, cells from stock culture are seeded on cell plates and cultured for about 18 hours to allow cell growth and attachment before starting the assay. On the first day of the assay, 5-(2,4-Dihydroxy-5-isopropyl-phenyl)-4-(4-morpholin-4-ylmethyl-phenyl)-isoxazole-3-carboxylic acid ethylamide is added to the medium at various concentrations up to 10 µ. Cells are cultured up to 72 or 96 hours and cell proliferation is determined using commercially available cell proliferation kits.

Table 1 shows the concentrations (nM) of 5-(2,4-Dihydroxy-5-isopropyl-phenyl)-4-(4-morpholin-4-ylmethyl-phenyl)-isoxazole-3-carboxylic acid ethylamide which inhibit cell proliferation by 50% (IC₅₀). The cells were continually exposed to 5-(2,4-Dihydroxy-5-isopropyl-phenyl)-4-(4-morpholin-4-ylmethyl-phenyl)-isoxazole-3-carboxylic acid ethylamide for either 72 or 96 hours and cell growth was determined by commercially available kits based on either SRB, Alamar blue, methylene blue or WST-1 methods.

**Table 1**

| **Tumor type** | **Cell line** | **IC₅₀ (nM)** |
|---|---|---|
| Breast | BT474 | 2.8 |
| | MDA-MB-361 | 6 |
| | MDA-MB-453 | 3.9 |
| | SKBr3 | 2.3 |
| | T47D | 2.6 |
| | MCF7 | 2.3 |
| | MDA-MB-231 | 7.7 |
| | MDA-MB-468 | 3.5 |
| Melanoma | SK-MEL-5 | 3 |
| | A375 | 3 |
| | MALME-3M | 7.7 |
| | SK-MEL-28 | 8 |
| | WM266.4 | 6.2 |
| Multiple myeloma (MM) | RPMI8226 | 36.7 |
| | U266 | 23.3 |
| Colon | BE | 2.8 |
| | Colo205 | 6.2 |
| | HCT116 | 16 |
| | HT29 | 30 |
| | MAWI | 50 |
| | RKO | 3.1 |
| Glioblastoma | U87MG | 6 |
| Head & Neck | HN5 | 8 |
| Leukemia | RPMI-8226 | 6.3 |
| Lung | A549 | 11.7 |
| | MV522 | 8.1 |
| | NCI-H1299 | 5.7 |
| | NCI-H460 | 14 |
| Ovarian | 41M | 3 |
| | A2780 | 6.1 |
| | CH1 | 2.8 |
| | SKOV3 | 3.7 |
| Prostate | PC3 | 5 |
| Stomach | NCI-N87 | 0.2 |
| Gastrointestinal stromal tumour (GIST) | MO7e | 10.6 |
| | GIST882 | 6.2 |
| | Baf3 | 22.4 |

### Example 2: In vitro effects of 5-(2,4-Dihydroxy-5-isopropyl-phenyl)-4-(4-morpholin-4-ylmethyl-phenyl)-isoxazole-3-carboxylic acid ethylamide on a panel of primary human tumor cells.

The anticancer activity of 5-(2,4-Dihydroxy-5-isopropyl-phenyl)-4-(4-morpholin-4-y)methylphenyl)-isoxazole-3-carboxylic acid ethylamide is evaluated in 30 human tumor xenografts *in vitro* using a clonogenic assay. In this assay, human cells derived from cancer patients are evaluated for the capacity of 5-(2,4-Dihydroxy-5-isopropyl-phenyl)-4-(4-morpholin-4-ylmethyl-phenyl)-isoxazole-3-carboxylic acid ethylamide to inhibit the formation of 3 dimensional colonies. These consist of tumor cells that possess the potential for anchorage independent growth in semisolid medium. The tumor xenografts which have never been cultured in cell culture plastic dishes are isolated from nude mice. Tumor cell suspensions are prepared and incubated in 24 well plates containing layers of soft agar. Under these conditions a special subpopulation of cells selectively grows to colonies. 5-(2,4-Dihydroxy-5-isopropyl-phenyl)-4-(4-morpholin-4-ylmethyl-phenyl)-isoxazole-3-carboxylic acid ethylamide was tested in 6 concentrations up to 10 µM. The tumor test panel comprises 1 to 6 models of 10 different human tumor or cancer types, which were bladder cancer, colon, liver, non small cell lung (adeno, squamous epithelium and large cell), small cell lung, mammary, ovary, pancreatic, melanoma and pleuramesothelioma. Antitumor effects are recorded as inhibition of colony formation in relation to untreated controls. The concentration which results in 50% reduction in colony formation (IC₅₀) are shown in Table 2. Further information on the method has been published (Burger et al., 2004; Fiebig et al., 2004; Smith et al., 2005).

Table 2 shows the concentration (nM) of 5-(2,4-Dihydroxy-5-isopropyl-phenyl)-4-(4-morpholin-4-ylmethyl-phenyl)-isoxazole-3-carboxylic acid ethylamide which inhibits colony formation by 50% (IC₅₀). The cells are continually exposed to 5-(2,4-Dihydroxy-5-isopropylphenyl)-4-(4-morpholin-4-ylmethyl-phenyl)-isoxazole-3-carboxylic acid ethylamide and colony formation is determined.

**Table 2**

| **Tumor Type** | **Tumor model** | **Histology** | **IC50 (nM)** |
|---|---|---|---|
| Bladder | BXF 1218 | Transitional cell carcinoma | 27 |
| | BXF 1228 | Transitional cell carcinoma | 630 |
| Colon | CXF 1103 | Adeno carcinoma | 13 |
| | CXF 158 | Adeno carcinoma | 369 |
| | CXF 1729 | Carcinoma | 467 |
| | CXF 1784 | Carcinoma | 418 |
| | CXF 609 | Adeno carcinoma | 55 |
| Liver | LIXF 575 | Hepatocellular carcinoma | 34 |
| Lung, non- small cell | LXFA 297 | Adeno carcinoma | 28 |
| | LXFA 526 | Adeno carcinoma | 5 |
| | LXFA 629 | Adeno carcinoma | 35 |
| | LXFA 983 | Adeno carcinoma | 126 |
| | LXFE 1422 | Squamous cell carcinoma | 48 |
| | LXFL 1647 | Large cell lung carcinoma | 34 |
| Lung, small cell | LXFS 615 | Small cell lung carcinoma | 30 |
| | LXFS 650 | Small cell lung carcinoma | 2 |
| Breast | MAXF 1162 | Invasive ductal carcinoma | 304 |
| | MAXF 1322 | Pap. adeno carcinoma | 29 |
| | MAXF 1384 | Adeno carcinoma | 209 |
| | MAXF 401 | Pap. adeno carcinoma | 78 |
| | MAXF 583 | Ductual adeno carcinoma | 333 |
| Melanoma | MEXF 1539 | Melanoma | 3 |
| | MEXF 462 | Amelanotic melanoma | 24 |
| | MEXF 535 | Amelanotic melanoma | 43 |
| | MEXF 672 | Amelanotic melanoma | 18 |
| | MEXF 989 | Amelanotic melanoma | 2 |
| Ovary | OVXF 1353 | Adeno carcinoma | 26 |
| | OVXF 1544 | Carcinoma | 53 |
| Pancreas | PAXF 1657 | Adeno carcinoma | 39 |
| Pleuramesothelioma | PXF 1118 | Biphasic pleuramesothelioma | 223 |

## Claims

1. 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for use in a method of the treatment of cancer of the colon, the liver, the pancreas, the stomach and/or the gastrointestinal tract.

2. 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for use according to claim 1 in a method of the treatment of cancer of the colon.

3. 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for use according to claim 1 in a method of the treatment of cancer of the liver.

4. 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for use according to claim 1 in a method of the treatment of cancer of the pancreas.

5. 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for use according to claim 1 in a method of the treatment of cancer of the stomach.

6. 5-(2,4-Dihydroxy-5-isopropyl-phenyl)-4-(4-morpholin-4-ylmethyl-phenyl)-isoxazole-3-carboxylic acid ethylamide or a tautomer thereof or a pharmaceutically acceptable salt or a hydrate or a solvate for use according to claim 1 in a method of the treatment of cancer of the gastrointestinal tract.

## Patentansprüche

1. 5-(2,4-Dihydroxy-5-isopropyl-phenyl)-4-(4-morpholin-4-ylmethyl-phenyl)-isoxazol-3-carbonsäureethylamid oder ein Tautomer davon oder ein pharmazeutisch verträgliches Salz oder ein Hydrat oder ein Solvat zur Verwendung in einem Verfahren zur Behandlung von Krebs des Dickdarms, der Leber, der Bauchspeicheldrüse, des Magens und/oder des Gastrointestinaltraktes.

2. 5-(2,4-Dihydroxy-5-isopropyl-phenyl)-4-(4-morpholin-4-ylmethyl-phenyl)-isoxazol-3-carbonsäureethylamid oder ein Tautomer davon oder ein pharmazeutisch verträgliches Salz oder ein Hydrat oder ein Solvat zur Verwendung nach Anspruch 1 in einem Verfahren zur Behandlung von Krebs des Dickdarms.

3. 5-(2,4-Dihydroxy-5-isopropyl-phenyl)-4-(4-morpholin-4-ylmethyl-phenyl)-isoxazol-3-carbonsäureethylamid oder ein Tautomer davon oder ein pharmazeutisch verträgliches Salz oder ein Hydrat oder ein Solvat zur Verwendung nach Anspruch 1 in einem Verfahren zur Behandlung von Krebs der Leber.

4. 5-(2,4-Dihydroxy-5-isopropyl-phenyl)-4-(4-morpholin-4-ylmethyl-phenyl)-isoxazol-3-carbonsäureethylamid oder ein Tautomer davon oder ein pharmazeutisch verträgliches Salz oder ein Hydrat oder ein Solvat zur Verwendung nach Anspruch 1 in einem Verfahren zur Behandlung von Krebs der Bauchspeicheldrüse.

5. 5-(2,4-Dihydroxy-5-isopropyl-phenyl)-4-(4-morpholin-4-ylmethyl-phenyl)-isoxazol-3-carbonsäureethylamid oder ein Tautomer davon oder ein pharmazeutisch verträgliches Salz oder ein Hydrat oder ein Solvat zur Verwendung nach Anspruch 1 in einem Verfahren zur Behandlung von Krebs des Magens.

6. 5-(2,4-Dihydroxy-5-isopropyl-phenyl)-4-(4-morpholin-4-ylmethyl-phenyl)-isoxazol-3-carbonsäureethylamid oder ein Tautomer davon oder ein pharmazeutisch verträgliches Salz oder ein Hydrat oder ein Solvat zur Verwendung nach Anspruch 1 in einem Verfahren zur Behandlung von Krebs des Gastrointestinaltraktes.

## Revendications

1. Ethylamide de l'acide 5-(2,4-dihydroxy-5-isopropyl-phényl)-4-(4-morpholin-4-ylméthyl-phényl)-isoxazole-3-carboxylique ou un tautomère de celui-ci ou un sel pharmaceutiquement acceptable ou un hydrate ou un solvate en vue d'une utilisation dans un procédé de traitement du cancer du côlon, du foie, du pancréas, de l'estomac et/ou du tractus gastro-intestinal.

2. Ethylamide de l'acide 5-(2,4-dihydroxy-5-isopropyl-phényl)-4-(4-morpholin-4-ylméthyl-phényl)-isoxazole-3-carboxylique ou un tautomère de celui-ci ou un sel pharmaceutiquement acceptable ou un hydrate ou un solvate en vue d'une utilisation selon la revendication 1 dans un procédé de traitement du cancer du côlon.

3. Ethylamide de l'acide 5-(2,4-dihydroxy-5-isopropyl-phényl)-4-(4-morpholin-4-ylméthyl-phényl)-isoxazole-3-carboxylique ou un tautomère de celui-ci ou un sel pharmaceutiquement acceptable ou un hydrate ou un solvate en vue d'une utilisation selon la revendication 1 dans un procédé de traitement du cancer du foie.

4. Ethylamide de l'acide 5-(2,4-dihydroxy-5-isopropyl-phényl)-4-(4-morpholin-4-ylméthyl-phényl)-isoxazole-3-carboxylique ou un tautomère de celui-ci ou un sel pharmaceutiquement acceptable ou un hydrate ou un solvate en vue d'une utilisation selon la revendication 1 dans un procédé de traitement du cancer du pancréas.

5. Ethylamide de l'acide 5-(2,4-dihydroxy-5-isopropyl-phényl)-4-(4-morpholin-4-ylméthyl-phényl)-isoxazole-3-carboxylique ou un tautomère de celui-ci ou un sel pharmaceutiquement acceptable ou un hydrate ou un solvate en vue d'une utilisation selon la revendication 1 dans un procédé de traitement du cancer de l'estomac.

6. Ethylamide de l'acide 5-(2,4-dihydroxy-5-isopropyl-phényl)-4-(4-morpholin-4-ylméthyl-phényl)-isoxazole-3-carboxylique ou un tautomère de celui-ci ou un sel pharmaceutiquement acceptable ou un hydrate ou un solvate en vue d'une utilisation selon la revendication 1 dans un procédé de traitement du cancer du tractus gastro-intestinal.
